# EUROPÄISCHE PATENTSCHRIFT

(11) **EP 1 332 736 B1**
(45) Veröffentlichungstag und Bekanntmachung des Hinweises auf die Patenterteilung: **04.05.2005**
(21) Anmeldenummer: 02024860.5
(22) Anmeldetag: 08.11.2002
(51) Int. Cl.: A61F 2/36

(54) **Schenkelhalsendoprothese**
Femoral neck endoprosthesis
Endoprothèse du col du fémur

(30) Priorität: 31.01.2002 DE 10204224
(43) Veröffentlichungstag der Anmeldung: 06.08.2003
(73) Patentinhaber: ESKA IMPLANTS GmbH & Co., 23526 Lübeck (DE)
(72) Erfinder: Grundei, Hans, Dr., 23558 Lübeck (DE); Thomas, Wolfram, Prof. Dr. med., 00135 Roma (IT); Benecke, Peter, Dr. med. habil., 23627 Gross Sarau (DE)
(74) Vertreter: Fuchs Mehler Weiss & Fritzsche

(56) Entgegenhaltungen:
- EP-A- 0 470 778
- EP-A- 0 791 342
- EP-A- 0 878 176
- WO-A-98/14141
- DE-U- 29 921 577
- FR-A- 2 225 141
- FR-A- 2 391 712
- FR-A- 2 578 738
- FR-A- 2 770 128
- GB-A- 764 600
- US-A- 4 846 841
- US-A- 5 413 610
- US-A- 6 096 084

## Beschreibung

Die Erfindung betrifft eine Schenkelhalsendoprothese als Teilimplantat für ein Hüftgelenk.

Als Teilimplantat wird nachfolgend ein in den Femur zu setzendes Implantat mit einer Gelenkkugel verstanden, das im Gegensatz zu einem Totalersatzimplantat für das Hüftgelenk, bei dem auch eine künstliche Gelenkpfanne in das natürliche, dann ausgefräste Acetabulum gesetzt wird, mit dem natürlichen Acetabulum mit dessen natürlicher Knorpelschicht zusammenarbeitet. Hierdurch ist es möglich, daß sich die Kugel näher an den anatomischen Größenverhältnissen orientiert. Von Interesse ist dies bei den auszuführenden Beugebewegungen.

Derartige Teilimplantate kommen nur dann zum Einsatz, wenn das natürliche Acetabelum noch soweit in Takt ist, daß es seine natürliche Funktion auszuüben vermag. Dabei ist ein besonderes Augenmerk auf die Beugefähigkeit des Gelenks aus natürlichem Acetabulum und künstlicher Gelenkkugel zu werfen. Kommt es aufgrund fehlerhafter Abstimmungen zu Mißverhältnissen, ist die Beugefähigkeit des Gelenkes stark eingeschränkt und es kommt zu einem Anschlag zwischen dem resezierten Schenkelhals und dem Hüftknochen.

Letztgenanntes Problem tritt insbesondere auf bei speziellen Schenkelhalsendoprothesen, wie sie im vorliegenden Falle von Interesse ist.

Aus der EP 0878176 ist beispielhaft eine Schenkhalsendoprothese bekannt, allerdings als Totalersatzimplantat für ein Hüftgelenk. Hieran wird sehr anschaulich, daß eine falsche Auswahl des Adapters für die Gelenkkugel nicht nur eine Einschränkung des Beugewinkels des Gelenkes nach sich führen kann, sondern auch zu einer Beinverlängerung.

Ein ähnliches Problem tritt auf bei einer Teilendoprothese wie im vorliegenden Fall. Erschwerend kommt hier noch hinzu, daß die Teilimplantate üblicherweise eine sogenannte Großkugel aufweisen, die sich den natürlichen Größenverhältnissen des natürlichen Acetabulums annähern.

Vor diesem Hintergrund ist es nun die Aufgabe der vorliegenden Erfindung eine Schenkelhalsendoprothese anzugeben, mit welcher die Anpassungsprobleme gelöst werden, bzw. ein Set für die Erstellung einer solchen Schenkelhalsendoprothese anzugeben, mit der der Operateur präoperativ oder in situ eine Anpassung an die natürlichen Größenverhältnisse des natürlichen Acetabulums vornehmen kann.

Die Lösung dieser Aufgabe gelingt mit einer Schenkelhalsendoprothese mit den Merkmalen des Anspruchs 1 und hinsichtlich des Sets gibt Anspruch 2 die notwendigen Merkmale an.

Hinsichtlich der Prothese als solches ist vorgesehen, daß diese eine in dem oberen Bereich eines Femurs unterhalb des Trochanter majors implantierbare Hülse aufweist, mit deren proximalen Ende ein Adapter in Form eines Doppelkonus zur Aufnahme einer Gelenkkugel verbindbar ist, wobei die Gelenkkugel eine konische Klemmhülse zur Verbindung mit dem Adapter aufweist, die in einer von einer Randeinfassung eingefassten topfförmigen Ausnehmung mündet.

Hintergrund dieser erfindungsgemäßen Ausbildung der Gelenkkugel ist folgender: Bei einer Schenkelhalsendoprothese wird der Schenkelhals im Gegensatz zur Vorbereitung des Femurs für die Implantation eines Langstielimplantates unter Belassung möglichst großer Bereiche natürlichen Knochens reseziert, daß heißt es bleibt in der Regel der ganze Schenkelhals zurück. Die topfförmige Ausnehmung in der Gelenkkugel dient nun dazu, über den Schenkelhalsstumpf geschoben zu werden, derart, daß die Randeinfassung den Schenkelhalsstumpf umgreift. Dabei ist die Gelenkkugel als sogenannte Großkugel ausgebildet, um - wie schon erwähnt - mit dem natürlichen Acetabulum zusammenarbeiten zu können.

Bemerkenswert hierbei ist, daß die Belastungskräfte nicht über die Randeinfassung und die topfförmige Ausnehmung in den Femur eingeleitet werden. Diese Aufgabe übernimmt der Adapter in Form eines Doppelkonus.

Die Gelenkkugel kann aus körperverträglichem Metall bestehen, aus einem Compound-Werkstoff oder einer Hartkeramik.

Hinsichtlich des Sets zur Erstellung der genannten Prothese ist vorgesehen, daß dieses Sets die implantierbare Hülse, den Adapter in Form eines Doppelkonus sowie wenigstens drei Gelenkkugeln umfaßt. Diese weisen jeweils unterschiedlich tief ausgeformte topfförmige Ausnehmungen auf. So kann standardgemäß vorgesehen sein, die Größen short, medium und long vorzusehen, was sich in direkter Weise auf die Beinlänge nach der Implantation auswirkt. Denkbar wäre es zwar auch, den Doppelkonus in seiner Länge zu verändern.

Um die Größenverhältnisse deutlich zu machen wird als Beispiel angegeben, daß der Schenkelhalsstumpf einen Durchmesser von 29 mm, die implantierbare Hülse im Bereich der Öffnung des Schenkelhalses hingegen einen Durchmesser von 25 mm haben kann. Der Kopf könnte beispielhaft einen Durchmesser von 36 - 48 mm aufweisen. Die Randeinfassung ist dann so dimensioniert, daß sie über den Schenkelhals geschoben werden kann.

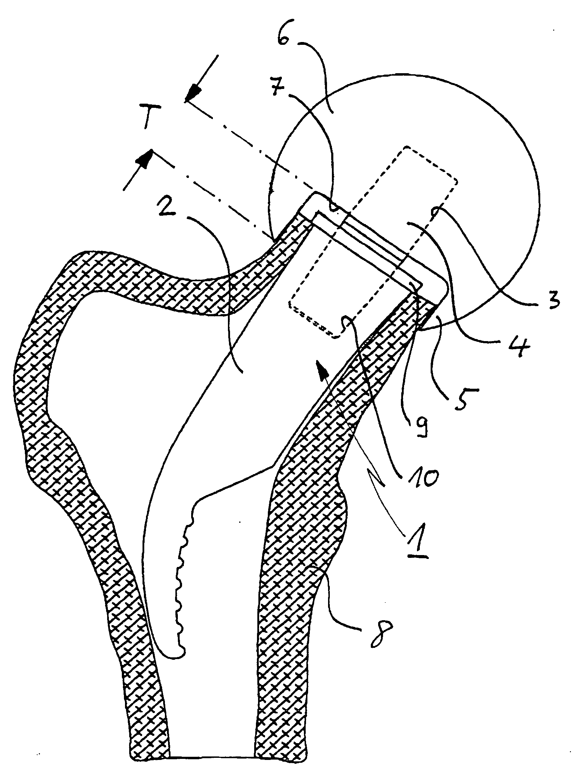

Die Erfindung wird beispielhaft anhand der einzigen Zeichnungsfigur näher erläutert. Hierbei zeigt:

In der Zeichnungsfigur ist ein Femur 8 schematisch im Schnitt gezeigt mit reseziertem Schenkelhals. In den Schenkelhals ist eine implantierbare Hülse 2 eingesetzt, wie sie beispielsweise in ähnlicher Weise aus der EP-0878176 bekannt ist. Diese Hülse kann mit einer offenmaschigen dreidimensionalen Raumnetzstruktur (nicht dargestellt) versehen sein oder aber als zementierbare Hülse ausgebildet sein, wie sie in der Zeichnungsfigur dargestellt ist. Aus diesem Grund trägt die dargestellte Hülse 2 auch einen Kragen 9, der sich auf dem Schenkelhalsstumpf abstützt. Im Falle einer zementlos zu implantierenden Hülse weist diese in der Regel keinen Kragen auf.

In der Hülse ist eine konische Klemmhülse 10 ausgebildet, in welche der Adapter 4 in Form eines Doppelkonus setzbar ist. Das Gegenstück findet sich in der Gelenkkugel 6 als konische Klemmhülse 3. Über den Adapter 4 ist somit die Gelenkkugel 6 mit der Hülse 2 dauerhaft, gleichwohl lösbar verbunden.

Die konische Klemmhülse 3 in der Gelenkkugel 6 mündet in einer topfförmigen Ausnehmung 7. Diese ist so dimensioniert, daß eine Randeinfassung 5 stehen bleibt, welche den Schenkelhalstumpf einfaßt. Die Belastungskräfte werden aber nicht über den Rand 5 in den Femur eingeleitet, sondern vielmehr über den Adapter 4.

In dem erfindungsgemäßen Set sind Gelenkkugeln 6 enthalten, die eine unterschiedliche Tiefe T der topfförmigen Ausnehmung 7 aufweisen. Hierdurch gelingt es dem Operateur, eine optimale Anpassung der Endoprothese vorzunehmen, insbesondere im Hinblick auf die Beinlänge.

## Patentansprüche

1. Schenkelhalsendoprothese (1) als Teilimplantat für ein Hüftgelenk, aufweisend
- einen Adapter (4) in Form eines Doppelkonus,
- eine Gelenkkugel (6),
- eine im oberen Bereich eines Femurs (8) unterhalb des Trochanter majors implantierbare Hülse (2), mit deren proximalen Ende (9) der Adapter zur Aufnahme der Gelenkkugel verbindbar ist bei der
- die Gelenkkugel eine konische Klemmhülse (3) zur Verbindung mit dem Adapter aufweist,
**dadurch gekennzeichnet,**
- **daß** die Klemmhülse in einer von einer Randeinfassung (5) eingefassten topfförmigen Ausnehmung (7) der Gelenkkugel mündet.

2. Set für die Erstellung einer Schenkelhalsendoprothese nach Anspruch 1, umfassend die implantierbare Hülse, den Adapter sowie wenigstens drei Stücke von der Gelenkkugel mit jeweils unterschiedlich tief ausgeformter topfförmiger Ausnehmung.

## Claims

1. Femoral neck endoprosthesis (1) as partial implant for a hip joint, having
- an adapter (4) in the form of a double cone,
- a joint head (6),
- a sleeve (2), which can be implanted in the upper region of a femur (8) below the trochanter major, with the proximal end (9) of which the adapter can be connected to receive the joint head, in which
- the joint head has a conical clamping sleeve (3) for connecting to the adapter,
**characterised in that**
- the clamping sleeve leads into a cup-shaped recess (7) of the joint head enclosed by an edge border (5).

2. Set for constructing a femoral neck endoprosthesis according to claim 1, comprising the implantable sleeve, the adapter and at least three pieces of the joint head having in each case cup-shaped recess shaped to different depth.

## Revendications

1. Endoprothèse de col du fémur (1) servant d'implant partiel pour une articulation de la hanche, présentant
- un adaptateur (4) sous la forme d'un cône double,
- une articulation orbiculaire (6),
- une gaine implantable (2) dans la partie supérieure du fémur (8) sous le trochanter majeur, dont l'extrémité proximale (9) peut être reliée à l'adaptateur pour accueillir l'articulation orbiculaire, où
- l'articulation orbiculaire présente une gaine conique de serrage (3) de liaison avec l'adaptateur,
**caractérisée par le fait que**
- la gaine de serrage débouche dans un évidement supérieur (7) de l'articulation orbiculaire entouré d'un rebord (5).

2. Ensemble pour la fabrication d'une endoprothèse de col du fémur selon la revendication 1, comprenant la gaine implantable, l'adaptateur ainsi qu'au moins trois pièces de l'articulation orbiculaire possédant chacune un évidement supérieur de profondeur différente.
